(19) Europäisches Patentamt
European Patent Office
Office européen des brevets

(11) Numéro de publication: **0 410 900 B1**

(12) **FASCICULE DE BREVET EUROPEEN**

(45) Date de publication de fascicule du brevet: **15.03.95**

(51) Int. Cl.$^6$: **C07C 271/28**

(21) Numéro de dépôt: **90420280.1**

(22) Date de dépôt: **13.06.90**

# (54) Procédé de préparation de méthylènedi (phényluréthane).

(30) Priorité: **29.06.89 FR 8909001**

(43) Date de publication de la demande:
**30.01.91 Bulletin 91/05**

(45) Mention de la délivrance du brevet:
**15.03.95 Bulletin 95/11**

(84) Etats contractants désignés:
**BE DE ES FR GB IT NL**

(56) Documents cités:
**EP-A- 0 016 441**
**EP-A- 0 038 005**
**EP-A- 0 216 273**
**GB-A- 2 004 866**

(73) Titulaire: **RHONE-POULENC CHIMIE**
**25, Ouai Paul Doumer**
**F-92408 Courbevoie Cédex (FR)**

(72) Inventeur: **Gubelmann, Michel**
**39, Boulevard des Belges**
**F-69002 Lyon (FR)**
Inventeur: **Rochin, Christophe**
**21, rue Dussaussoy**
**F-69006 Lyon (FR)**
Inventeur: **Allandrieu, Christian**
**8, Avenue Salvador Allende**
**F-69100 Villeurbanne (FR)**

(74) Mandataire: **Dubruc, Philippe et al**
**RHONE-POULENC CHIMIE,**
**Direction de la Propriété Industrielle,**
**25, Ouai Paul Doumer**
**F-92408 Courbevoie Cédex (FR)**

**EP 0 410 900 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne un procédé de préparation de méthylènedi(phényluréthane). Elle a plus particulièrement trait à la préparation de méthylènedi(phényluréthane) par condensation d'un N-phénylcarbamate d'alkyle et d'un agent méthylènant.

Le méthylènedi(phényluréthane) couramment appelé MDU est un intermédiaire utile à la fabrication du méthylènedi(phénylisocyanate) connu sous l'appelation MDI. En effet, le MDU peut être pyrolysé en MDI de manière en soi connue. Le MDI est utile notamment on tant que matière première pour les mousses et élastomères de polyuréthanes.

Le MDI est fabriqué conventionnellement par phosgénation de la diamine qui résulte de la condensation de l'aniline et du formaldéhyde. Le produit commercial est un mélange des divers isomères du MDI et d'oligomères couramment désignés par poly-méthylènedi(phénylisocyanate) PMDI, dont le MDI pur est susceptible d'être isolé.

Pour des raisons évidentes liées à la toxicité du phosgène et aux inconvénients associés a la génération d'acide chlorhydrique lors de l'étape de phosgénation, de nombreuses recherches ont été entreprises aux fins de proposer des voies d'accès au MDI ne nécessitant pas d'étape de phosgénation.

C'est ainsi qu'il a été proposé divers procédés de préparation du MDI a partir de N-phénylcarbamates d'alkyle comprenant une première étape de condensation du N-phénylcarbamate avec du formaldéhyde pour former un mélange renfermant du diphénylméthanedicarbamate et des poly-méthylènedi-(phénylcarbamate), homologues supérieurs des méthylènedi(phénylcarbamate) (ou MDU), suivie d'une étape de décomposition thermique.

L'un des inconvénients présenté par ce type de procédés réside dans le fait que la proportion de MDI dinucléaires et, en particulier, de l'isomère 4,4' est insuffisante.

Un autre inconvénient présenté par ce type de procédés réside dans le fait que lors de l'étape de condensation il se forme aux côtés du diphénylméthanedicarbamate souhaité des proportions notables de composés tels les N-carboalcoxyanilinophénylméthanes, les bis(N-carboalcoxyanilino)méthanes, et les N,N'-dicarboalcoxyaminobenzylanilines et leurs dérivés de condensation supérieure.

Ces diverses impuretés sont gênantes pour la conversion du mélange réactionnel en diisocyanates recherchés.

Dans le brevet américain n° 4,146,727, il a été proposé de réarranger des impuretés de type N-benzylique de formule (I)

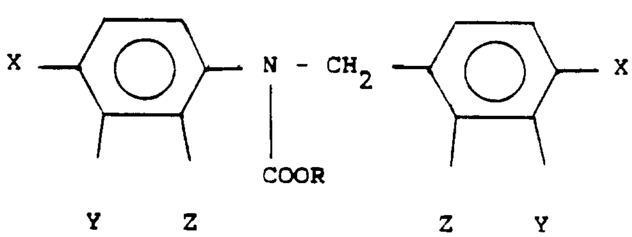

(I)

dans laquelle X, Y ou Z peuvent notamment représenter un groupe - NHCOOR, R est un groupe alkyle comportant de 1 à 3 atomes de carbone, leurs dimères, trimères, tétramères, etc, en diphénylméthanedicarbamate par leur mise en contact à une température comprise entre 50 et 170 ° C, de préférence entre 80 et 130 ° C avec une quantité catalytique d'un milieu acide protonique fort.

Il résulte de l'enseignement de ce document qu'une étape supplémentaire devrait être mise en oeuvre pour supprimer au moins partiellement les dites impuretés.

Dans la demande de brevet français n° 2 460 972 (correspondant au brevet américain n° 4 319 018) il est proposé de réaliser l'étape de condensation du N-phénylcarbamate d' alkyle et du formaldéhyde ou d'une substance générant du formaldéhyde en présence simultanée d'au moins un composé qui peut notamment être choisi parmi les bis(N-carboalcoxyanilino) méthanes et les N,N'-dicarboalcoxyaminobenzylanilines et d'une solution aqueuse acide dont la concentration est ajustée de telle sorte que la cinétique de réaction soit acceptable et que les réactions secondaires soient maintenues à un niveau minimal, à une température comprise entre 10 et 150 ° C et de préférence entre 20 et 120 ° C.

Le procédé décrit dans cette demande ne permet pas d'éliminer totalement les impuretés en cause.

Dans le brevet européen EP 16 441, il est décrit un procédé de préparation de méthyl di-(phénylcarbamate) par condensation d'esters d'acide N-aryl carbamique avec un agent méthylénant tel que

le formaldéhyde. La réaction est réalisée en présence d'un catalyseur choisi parmi les acides minéraux, sulfoniques ou acides de Lewis, et en présence d'un solvant, dont la constante diélectrique, mesurée à 20 °C, est d'au moins 20. Cependant, la sélectivité, le rendement en méthyl di(phénylcarbamate) restent faibles.

Le brevet européen EP 38 005 décrit le même type de réaction mais le catalsyeur utilisé est un acide carboxylique dont le pK est d'au plus 4, mais la proportion en composés comprenant 3 noyaux aromatiques est élevée.

Il est par ailleurs connu, avec le brevet européen EP 216 273, de réaliser la condensation d'esters d'acide N-aryl carbamique avec un agent méthylénant, en présence d'un acide minéral en tant que catalyseur. L'inconvénient de ce procédé est qu'il est mis en oeuvre en deux étapes.

Il serait très souhaitable de pouvoir disposer d'un procédé en un seul stade, de préparation de méthylènedi(phényluréthane) par condensation d'un N-phénylcarbamate d'alkyle et d'un agent méthylènant avec une sélectivité améliorée en méthylènedi(phényluréthane). Il serait également souhaitable de pouvoir disposer d'un procédé permettant de contrôler la proportion d'isomère-4,4' dans le produit difonctionnel.

La présente invention a donc pour objet un procédé de préparation de méthylènedi(phényluréthane) par condensation d'un N-phénylcarbamate d'alkyle et d'un agent méthylènant en présence d'un acide protonique caractérisé en ce que l'acide protonique est l'acide fluorhydrique.

Les N-phénylcarbamates d'alkyle utilisables dans le cadre du présent procédé sont ceux dont le groupe alkyle (ou cycloalkyle) renferme de 1 à 6 et, de préférence de 1 à 4 atomes de carbone. Le N-phénylcarbamate d'éthyle convient plus particulièrement bien à la mise en oeuvre du présent procédé.

Par agent méthylènant, on entend dans le cadre du présent procédé, le formaldéhyde ou des composés capables de libérer du formaldéhyde dans les conditions de la réaction tels le paraformaldéhyde, le trioxanne, les dialcoxyméthanes, en particulier le méthylal, et l'urotropine (hexaméthylènetétramine). De préférence, on recourt au formaldéhyde, au paraformaldéhyde, au trioxanne ou au méthylal.

La stoéchiométrie de la réaction de condensation pour former le méthylènedi(phényluréthane) implique la présence de 2 moles de N-phénylcarbamate d'alkyle par mole de groupements méthylène($-CH_2-$). Il est avantageux d'engager le N-phénylcarbamate d'alkyle en excès par rapport à la stoéchiométrie sans qu'il soit utile que le rapport molaire N-phénylcarbamate d'alkyle/$-CH_2-$ excède 10. Ce rapport est de préférence compris entre 3 et 7.

Le procédé selon l'invention requiert la présence d'acide fluorhydrique. Il est avantageux de mettre en oeuvre de l'acide fluorhydrique anhydre. Lorsque l'agent méthylénant est le formaldéhyde, le trioxanne ou le paraformaldéhyde, il se produit lors de la réaction de condensation de l'eau qui peut être gênante pour la récupération de l'acide fluorhydrique, sans nuire pour autant à la réaction en cause.
Lorsque l'agent méthylènant est un dialcoxyméthane il se produit lors de la réaction de condensation, un alcool, inerte dans les conditions de la réaction et qui peut être aisément séparé de l'acide fluorhydrique.

La quantité d'acide fluorhydrique à mettre en oeuvre n'est pas critique. L'acide fluorhydrique peut être engagé en quantité importante par rapport aux réactifs de telle sorte qu'il constitue le solvant dans le milieu réactionnel. L'acide fluorhydrique peut être présent en quantité plus faible.

Pour une bonne mise en oeuvre de l'invention le rapport molaire de l'acide fluorhydrique au N-phénylcarbamate d'alkyle est au moins égal à 5 et, de préférence, il est inférieur à 20.

La température de réaction est en générale comprise entre - 20° et 80°C.

Pour une bonne mise en oeuvre du présent procédé la température sera comprise entre 0 et 60°C. En effet, au delà de 60°C on observe à la fois une diminution de la proportion d'isomère 4,4' du méthylènedi-(phényluréthane), une augmentation en dérivés porteurs de 3 noyaux aromatiques, et une isomérisation de l'isomère 4,4' en isomère 2,4'.

On observe également, en dessous de 60°C, que la proportion de dérivés porteurs de 3 noyaux aromatiques reste faible dans le mélange réactionnel et que plus la température est basse plus la proportion d'isomère 4,4' recherché est importante dans le méthylènedi(phényluréthane) produit.

La pression n'est pas un paramètre essentiel du procédé. Toutefois, lorsque la température de réaction dépasse 20°C il est préférable d'opérer sous une pression supérieure à la pression atmosphérique pour maintenir l'acide fluorhydrique sous forme liquide.

La procédé selon l'invention peut être mis en oeuvre dans l'acide fluorhydrique comme solvant ou dans un mélange d'acide fluorhydrique et d'un solvant organique. A titre d'exemples de solvants organiques utilisables dans le cadre du présent procédé on peut citer : des hydrocarbures aliphatiques tels l'hexane et l'heptane ; des hydrocarbures alicycliques tels le cyclohexane et le méthylcyclohexane ; et les hydrocarbures halogénés tels le chloroforme, le chlorure de méthylène, le chlorure d'éthylène, le chlorocyclohexane, le perchlorocyclohexane, le chlorobenzène et le dichlorobenzène. Quand on utilise un tel solvant, il représente au maximum 300 % et, de préférence, de 10 à 150 % en poids par rapport au N-phénylcarbamate d'alkyle

engagé. La durée de réaction peut varier dans de larges limites ; elle est généralement comprise entre 15 mn et 8 heures.

La réaction peut être mise en oeuvre en discontinu ou en continu.

En fin de réaction ou du temps imparti à celle-ci on récupère le produit recherché par tous moyens appropriés, par exemple par évaporation de l'acide fluorhydrique.

Les exemples ci-après illustrent la présente invention.

Les conventions suivantes y sont utilisées :

- MDU RR(%) : représente le rendement en méthylène di(carbanilate d'éthyle) calculé par rapport au nombre de mole initiales de groupes $-CH_2-$.
- 3-Ph(%) : représente le rendement (RR) en composés présentant 3 noyaux aromatiques calculé de manière analogue
- 4,4' : représente le méthylène-4,4' di(carbanilate d'éthyle) de formule :

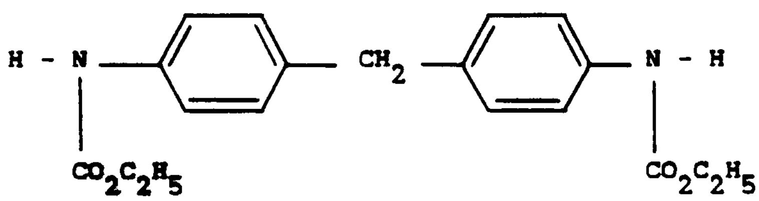

- 2,4' : représente le méthylène-2,2' di(carbanilate d'éthyle)

. A représente le bis(N-carboéthoxyanilino)méthane de formule

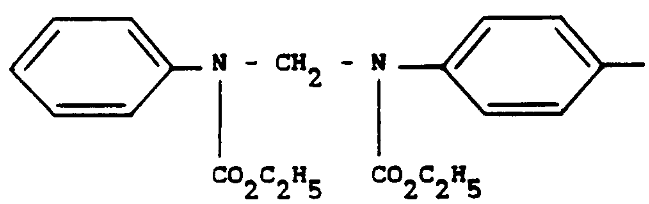

. B représente les N,N'-dicarboéthoxyaminobenzylanilines de formule :

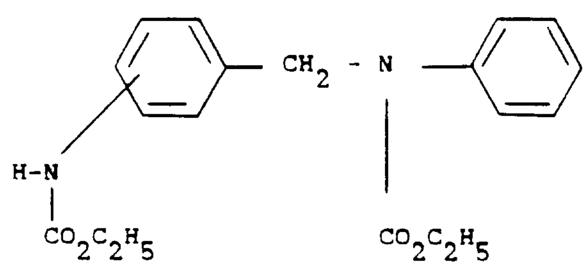

EXEMPLE 1 à 3 :

Dans un réacteur en Hastelloy de 50 cm3 de capacité, muni d'une agitation magnétique, on charge :

50 mmol de phénylcarbamate d'éthyle,

2 mmol de trioxanne (soit 6 mmol de groupements $-CH_2-$),

20 cm3 (1 mol) d'acide de fluorhydrique anhydre ou x cm3 de chlorure de méthylène et (20-x) cm3 d'acide fluorhydrique anhydre.

En fin d'essais on analyse le mélange réactionnel par chromatographies en phase gazeuse et en phase liquide.

Les conditions particulières ainsi que les résultats obtenus en 2 heures de réaction à 40°C figurent dans le tableau (I) ci-après :

TABLEAU I

| Ex. N° | HF cm3 | $CH_2Cl_2$ cm3 | MDU RR(%) | Répartition isomérique (%) | | 3-Ph (%) |
|---|---|---|---|---|---|---|
| | | | | - 4,4' | - 2,4' | |
| 1 | 20 | 0 | 81,5 | 90 | 10 | 0 |
| 2 | 10 | 10 | 97,7 | 91 | 9 | 2,5 |
| 3 | 5 | 15 | 97,7 | 91 | 9 | 2 |

Dans ces exemples on ne détecte pas la présence d'impuretés comportant une liaison méthylèneamino.

{RR(A) = RR(B) = 0}

EXEMPLES 4 ET 5 :

On reproduit l'exemple 1 ci-avant en ne modifiant que la température de réaction.
Les conditions particulière ainsi que les résultats obtenus figurent dans le tableau (II) ci-après :

TABLEAU II

| Ex. N° | T° C | MDU RR(%) | Répartition isomérique | | . 3-Ph (%) |
|---|---|---|---|---|---|
| | | | - 4,4' | - 2,4' | |
| 1 | 40 | 81,5 | 90 | 10 | 0 |
| 4 | 20 | 80 | 94 | 6 | 0 |
| 5 | 0 | 75 | 96 | 4 | 0 |

Dans ces exemples on ne détecte pas la présence d'impuretés comportant une liaison méthylèneamino.

{RR(A) = RR(B) = 0}

EXEMPLE 6 :

On reproduit l'exemple 4 ci-avant en remplaçant le trioxanne par une quantité équivalente de groupements méthylène sous forme de diméthoxyméthane-(6mmol).
Les résultats sont les suivants :
MDU RR(%) : 83

| Répartition isomérique (%) | |
|---|---|
| - 4,4' | 94 |
| - 2,4' | 6 |
| - 3-Ph (%) | 0 |

Dans cet exemple on ne détecte pas la présence d'impuretés comportant une liaison méthylèneamino

{RR(A) = RR(B) = 0}

## Revendications

**1.** Procédé de préparation de méthylènedi(phényluréthane) par condensation d'un N-phénylcarbamate d'alkyle et d'un agent méthylènant en présence d'un acide protonique caractérisé en ce que l'acide protonique est l'acide fluorhydrique.

2. Procédé selon la revendication 1, caractérisé en ce que la réaction est conduite en phase liquide.

3. Procédé selon la revendication 1 ou 2, caractérisé en ce que le rapport molaire N-phénylcarbamate d'alkyle/-$CH_2$- est compris entre 3 et 7.

4. Procédé selon l'une quelconque des revendications précédentes, caractérisé en ce que l'agent méthylénant est choisi parmi le formaldéhyde, le paraformaldéhyde, le trioxanne et le méthylal.

5. Procédé selon l'une quelconque des revendications précédentes, caractérisé en ce que le N-phénylcarbamate d'alkyle est le N-phénylcarbamate d'éthyle.

6. Procédé selon l'une quelconque des revendications précédentes, caractérisé an ce que la réaction est conduite également en présence d'un solvant organique.

7. Procédé selon la revendication 6, caractérisé en ce que le solvant organique est le chlorure de méthylène.

8. Procédé selon l'une quelconque des revendications précédentes, caractérisé en ce que le rapport molaire HF/N-phénylcarbamate d'alkyle est supérieur ou égal à 5.

9. Procédé selon l'une quelconque des revendications précédentes, caractérisé en ce que le rapport molaire HF/N-phénylcarbamate d'alkyle est inférieur ou égal à 20.

10. Procédé selon l'une quelconque des revendications précédentes, caractérisé en ce que la température de réaction est comprise entre 0 et 60 °C.

**Claims**

1. Process for the preparation of methylenedi(phenylurethane) by condensation of an alkyl N-phenylcarbamate and a methylenating agent in the presence of a protic acid, characterized in that the protic acid is hydrofluoric acid.

2. Process according to claim 1, characterized in that the reaction is carried out in the liquid phase.

3. Process according to claim 1 or 2, characterized in that the alkyl N-phenylcarbamate/-$CH_2$- molar ratio is between 3 and 7.

4. Process according to any one of the preceding claims, characterized in that the methylenating agent is chosen from formaldehyde, paraformaldehyde, trioxane and methylal.

5. Process according to any one of the preceding claims, characterized in that the alkyl N-phenylcarbamate is ethyl N-phenylcarbamate.

6. Process according to any one of the preceding claims, characterized in that the reaction is also carried out in the presence of an organic solvent.

7. Process according to claim 6, characterized in that the organic solvent is methylene chloride.

8. Process according to any one of the preceding claims, characterized in that the HF/alkyl N-phenylcarbamate molar ratio is greater than or equal to 5.

9. Process according to any one of the preceding claims, characterized in that the HF/alkyl N-phenylcarbamate molar ratio is less than or equal to 20.

10. Process according to any one of the preceding claims, characterized in that the reaction temperature is between 0 and 60 °C.

## Patentansprüche

1. Verfahren zur Herstellung von Methylendi(phenylurethan) durch Kondensation von einem Alkyl-N-phenylcarbamat und einem Methylierungsmittel in Gegenwart einer Protonensäure, dadurch gekennzeichnet, daß die Protonensäure Flußsäure ist.

2. Verfahren nach Anspruch 1, dadurch gekennzeichnet, daß die Reaktion in flüssiger Phase durchgeführt wird.

3. Verfahren nach Anspruch 1 oder 2, dadurch gekennzeichnet, daß das molare Verhältnis von Alkyl-N-phenylcarbamat zu $-CH_2-$ zwischen 3 und 7 liegt.

4. Verfahren nach einem der vorhergehenden Ansprüche, dadurch gekennzeichnet, daß das Methylierungsmittel gewählt ist aus Formaldehyd, Paraformaldehyd, Trioxan und Methylal.

5. Verfahren nach einem der vorhergehenden Ansprüche, dadurch gekennzeichnet, daß das Alkyl-N-phenylcarbamat Ethyl-N-phenylcarbamat ist.

6. Verfahren nach einem der vorhergehenden Ansprüche, dadurch gekennzeichnet, daß die Reaktion gleichermaßen in Gegenwart eines organischen Lösemittels durchgeführt wird.

7. Verfahren nach Anspruch 6, dadurch gekennzeichnet, daß das organische Lösemittel Methylenchlorid ist.

8. Verfahren nach einem der vorhergehenden Ansprüche, dadurch gekennzeichnet, daß das molare Verhältnis von HF zu Alkyl-N-phenylcarbamat größer oder gleich 5 ist.

9. Verfahren nach einem der vorhergehenden Ansprüche, dadurch gekennzeichnet, daß das molare Verhältnis von HF zu Alkyl-N-phenylcarbamat kleiner oder gleich 20 ist.

10. Verfahren nach einem der vorhergehenden Ansprüche, dadurch gekennzeichnet, daß die Reaktionstemperatur zwischen 0 und 60 °C liegt.